# EUROPEAN PATENT SPECIFICATION

(11) **EP 3 269 311 B1**
(45) Date of publication and mention of the grant of the patent: **02.12.2020**
(21) Application number: 17181054.2
(22) Date of filing: 12.07.2017
(51) Int. Cl.: A61B 17/00, A61B 17/32

(54) **TENDON STRIPPER**
SEHNEN-ABTRENNVORRICHTUNG
SÉPARATEUR DU TENDON

(30) Priority: 15.07.2016 US 201615211683
(43) Date of publication of application: 17.01.2018
(73) Proprietor: Arthrex Inc, Naples, FL 34108-1945 (US)
(72) Inventor: WEBER, Robert M., Chino Hills, CA 91709 (US); JOLLY, Jacob A., Naples, FL 34110 (US)
(74) Representative: Lohr, Jöstingmeier & Partner

(56) References cited:
- EP-A1- 2 779 885
- WO-A1-2016/103900
- WO-A2-2006/119238
- US-A- 6 045 561
- US-A- 6 110 190

## Description

### BACKGROUND

This disclosure relates to a tendon stripper for use in separating at least a portion of a tendon from adjacent muscle tissue.

Tendon strippers are used to harvest tendons for use in orthopedic procedures. In one known procedure, a tendon stripper is used to harvest at least a portion of a hamstring. The harvested hamstring tendon may then be used in an anterior cruciate ligament (ACL) reconstruction surgery, for example. Tendons are also known to be harvested from other areas of the body, such as the wrist, and can used in other types of orthopedic procedures, such as ulna collateral ligament (UCL) reconstructions.

WO 2016/103900 A1 discloses a medical instrument for gripping a wire having a cross-sectional shape having a curved surface. EP 2779 885 A1 discloses an operational device especially intended to proceed with an operation inside the body of a living being. This device comprises a control handle and a flexible element provided with a proximal end connected to this control handle. The flexible element comprises at a distal end, a steerable area comprising a rigid head. The control handle comprises a control lever arranged for controlling said steerable area of this flexible element. The flexible element comprises at its distal end, a retractable tool in form of a hook, and this hook being arranged to be operated by said control handle.

A document describing a surgical tool configured to aid in performing a procedure of detaching an internal mammary artery and the like is known from US 6,110,190.

Another document disclosing a surgical tool for tissue eviscerator having a cauteric electric end to cauterize the surrounding tissue is known from WO 2006/119238.

A further document disclosing a surgical knot manipulator device for facilitating tying and untying of surgical knots is known from US 6,045,561.

### SUMMARY

The present invention is defined by the features of the independent claim. Preferred embodiments are given in the dependent claims. This disclosure relates to a tendon stripper, which may be used to separate at least a portion of a tendon from adjacent muscle tissue. The harvested tendon can then be used in various orthopedic procedures, such as ACL and UCL reconstructions, to name a few examples.

### BRIEF DESCRIPTION OF THE DRAWINGS

Figure 1 illustrates an example tendon stripper with a retention structure in a closed position.
Figure 2 illustrates the example tendon stripper with the retention structure in an open position.
Figure 3 is a side view of the tendon stripper of Figures 1-2 with the retention structure in the open position, and illustrates the detail of the retention structure and a tip of the tendon stripper.
Figure 4 is a perspective view of the tendon stripper of Figures 1-2, and illustrates the detail of the retention structure and the tip.
Figure 5 is a side view of the tendon stripper of Figures 1-2 with the retention structure in the closed position, and illustrates the detail of the retention structure and the tip.
Figure 6A is a cross-sectional view taken along line 6A-6A from Figure 5, and illustrates a first example tip configuration.
Figure 6B is a cross-sectional view taken along line 6B-6B from Figure 5, and illustrates a second example tip configuration
Figure 7 illustrates the tendon stripper of Figures 1-2 in use.
Figure 8 is a side view of another example tendon stripper. In this example, the retention structure has a straight leading surface.

### DETAILED DESCRIPTION

A method according to an exemplary aspect of the present disclosure includes, *inter alia,* capturing a tendon in a retention structure of a tendon stripper, and moving the tendon stripper distally to separate at least a portion of a tendon from adjacent muscle tissue. The tendon stripper includes a shaft with a tip at a distal end of the shaft. The tip tapers to a point, which preferably increases the ease of separating tendon from adjacent muscle.

In a further example, the point of the tip is provided at a junction between the tendon and the muscle tissue during the separating step, and the point of the tip is coincident with a centerline of the shaft.

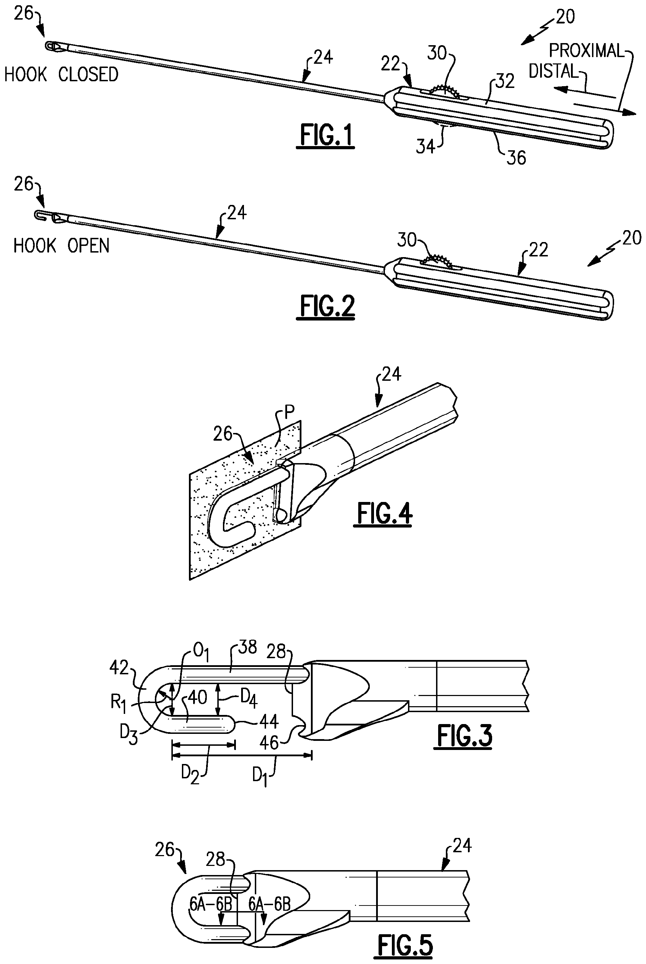

Figure 1 illustrates an example tendon stripper 20. The tendon stripper 20 includes a handle 22, a shaft 24, and a retention structure. In the figures, the retention structure is a hook 26. This disclosure is not limited to tendon strippers with hooks, however, and extends to other types of retention structures such as squares, loops, corkscrews, and slotted sleeves, to name a few examples.

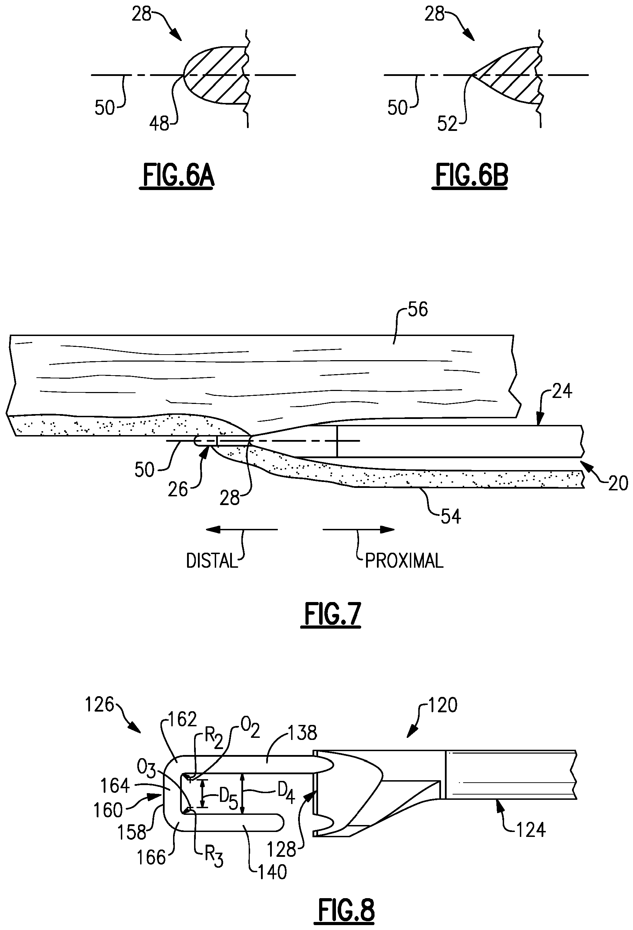

The shaft 24 projects from the handle 22 in a distal direction. The hook 26 is moveable relative to the shaft 24 between an open position (Figure 2) in which a tendon 54 (Figure 7) can be captured by the hook 26, and a closed position (Figure 1) in which the captured tendon 54 is secured relative to a tip 28 of the shaft. As will be explained below, the tip 28 is configured to separate the tendon 54 from adjacent muscle tissue 56 (Figure 7).

In this example, the hook 26 is selectively moveable between the open and closed positions by movement of a trigger 30 provided on the handle 22. In this example, the trigger 30 is configured for movement by a user's (e.g., a surgeon's) thumb. The trigger 30 is moveable in the distal and proximal directions to move the hook between the open and closed positions, respectively. The "proximal" and "distal" directions are labeled across the figures for purposes of explanation only. In some examples, the trigger 30 is moveable to intermediate positions between the open and closed positions to accommodate different tendon sizes, for example. Further, in one example, the trigger 30 is mechanically biased to the closed position.

While in the disclosed embodiment the hook 26 is moveable relative to the shaft 24, in other examples the shaft 24 could be moveable relative to the hook 26. In that case, the shaft 24 would be selectively moveable in response to movement of the trigger 30.

The trigger 30 in this example is provided on a superior, or top, surface 32 of the handle 22. The handle 22 may also include a second trigger 34 (shown in phantom in Figure 1) on an inferior, or bottom, surface 36 of the handle 22. The first trigger 30 and the second trigger 34, if present, are both configured to selectively move the hook 26 between the open and closed positions. In one example, the second trigger 34 moves with the first trigger 30. Alternatively, the second trigger 34 could move independently of the first trigger 30.

The arrangement of the hook 26 allows for increased ease of capturing a tendon within the hook 26. Further, the hook 26 and tip 28 arrangement provides for increased functionality of the tendon stripper 20. Namely, the arrangement allows the tendon stripper 20 to be easily used on both sides of a patient's body, and by either hand of a user (e.g., a surgeon).

With reference to Figure 3, in one example the hook 26 is substantially U-shaped. In particular, the hook 26 includes a first linear portion 38, a second linear portion 40, and a curved portion 42 between the first and second linear portions 38, 40. The first and second linear portions 38, 40 extend in a linear direction along substantially straight lines, and are substantially parallel to one another along their entire respective lengths D₁, D₂.

In this example, the first linear portion 38 has a length D₁ greater than the length D₂ of the second linear portion 40. Further, the first linear portion 38 is received at least partially inside the shaft 24, and is mechanically coupled to the trigger 30. The first linear portion 38 may be directly coupled to the trigger 30, or the tendon stripper 20 may include an intermediate actuator, such as a shaft or cable, configured to transmit force from the trigger 30 to the first linear portion 38.

The proximal end 44 of the second linear portion 40 is configured to be received in a socket 46 provided adjacent the tip 28. When in the open position, as shown in Figure 3, a tendon can be captured within the hook 26 by virtue of the space between the proximal end 44 of the second linear portion 40 and the tip 28.

In this example, the curved portion 42 follows an arc defined by a circle having a center at an origin O₁ between the first and second linear portions 38, 40. The circle has a radius R₁ originating at origin O₁ and a diameter D₃ passing through origin O₁. The curved portion 42 provides a semi-circle, and in this example makes a 180° bend about the origin O₁ to connect the first and second linear portions 38, 40. Further, the first and second linear portions 38, 40 are spaced-apart from one another by a distance D₄ substantially equal to the diameter D₃. Spacing the first and second linear portions 38, 40 apart from one another by the distance D₄ increases the ease of capturing a tendon within the hook 26. Further, the tip 28 spans the entire distance D₄ between the first and second linear portions 38, 40, which increases the available cutting surface.

With reference to Figure 4, the hook 26 is provided entirely in a common plane P, in one example, and the hook 26 is bisected by the common plane P. That is, the first and second linear portions 38, 40 and the curved portion 42 are entirely provided in a common plane P. The first and second linear portions 38, 40 and the curved portion 42 do not bend out of the common plane P in the side-to-side directions (e.g., in or out of the page, relative to Figure 4). In one example, the common plane P contains a centerline 50 (Figures 6A-6B) bisecting the tendon stripper 20. This arrangement provides the hook 26 with symmetry, which makes the tendon stripper 20 easy to use on either side of a patient's body, and by either hand of the user. To this end, the trigger 30 and the second trigger 34 (if present) are also provided in the common plane P such that the triggers 30, 34 are easily accessed by the user when the tendon stripper 20 is held in various orientations.

Figure 5 illustrates the hook 26 in a closed position, in which the hook 26 and the tip 28 enclose a space for receiving a tendon 54. In one example, the tip 28 is provided along a centerline 50 (Figures 6A-6B) of the shaft 24. Additionally, the tip 28 may taper to a point. The tip 28 thus increases the ease of separating a tendon from adjacent muscle tissue.

In one example, illustrated in Figure 6A, the tip 28 tapers to a rounded point 48. The rounded point 48 is provided on a centerline 50 of the shaft 24. The centerline 50 of the shaft 24 in one example is also a centerline of the tendon stripper 20, and is contained within the common plane P. In another example, the tip 28 tapers to a sharp point 52 (Figure 6B). While the rounded point 48 is blunt (i.e., not sharp), the tip 28 still increases the ease of separating tendon from adjacent muscle tissue because the tip 28 is provided along the centerline 50.

Figure 7 schematically illustrates the tendon stripper 20 in use. In Figure 7, the hook 26 has captured a tendon 54. A user separates the tendon 54 from adjacent muscle tissue 56 by movement of the tendon stripper 20 in the distal direction. As shown in Figure 7, the arrangement of the hook 26 and tip 28 is such that the point (e.g., points 48, 52) of the tip 28 is provided at a junction between the tendon 54 and the muscle tissue 56. With reference to the orientation of Figure 7, the tendon 54 is below the centerline 50 proximal of the tip 28, and the muscle tissue 56 is above the centerline 50 proximal of the tip 28. The hook 26 of the present disclosure is configured to maintain the position of the tip 28 at the junction between the tendon 54 and the muscle tissue 56, while also allowing for ease of movement of the tendon stripper 20 in the distal direction.

Figure 8 is a side view of another example tendon stripper 120, which is not part of the invention. To the extent not otherwise described or shown, the tendon stripper 120 corresponds to the tendon stripper 20 of Figures 1-7, with like parts preappended with a "1."

In Figure 8, the tendon stripper 120 includes a retention structure with a straight leading edge 158. In this example, the retention structure is a hook 126. Again, this disclosure is not limited to hooks and extends to other types of retention structures.

The hook 126 in this example includes first and second linear portions 138, 140, which are substantially similar to the first and second linear portions 38, 40 of the hook 26. In the hook 126, the first and second linear portions 138, 140 are connected by a connection section 160 having a first curved portion 162, a straight portion 164 providing the straight leading edge 158, and a second curved portion 166.

In this example, the first curved portion 162 extends between the first linear portion 138 and the straight portion 164. The first curved portion 162 follows a radius R₂ of curvature. The radius R2 extends from an origin O₂ between the first and second linear portions 138, 140, but substantially closer to the first linear portion 138 than the second linear portion 140. In this example, the first curved portion 162 bends 90° about the origin O₂.

The straight portion 164 in this example extends substantially perpendicular to the first and second linear portions 138, 140. The straight portion 164 provides the tendon stripper 120 with a straight leading edge 158, which facilitates movement of the tendon stripper 120 within a patient's body.

The second curved portion 166 connects the straight portion 164 and the second linear portion 140. The second curved portion 164 is shaped similar to the first curved portion 162, and in particular follows a radius R₃ of curvature, which extends from an origin O₃. The origin O₃ is between the first and second linear portions 138, 140, but is substantially closer to the second linear portion 140 than the first linear portion 138. In this example, the second curved portion 166 bends 90° about the origin O₃.

In this example, the straight portion 164 extends a distance D₅ between the first and second curved portions 162, 166. The distance D₅ is equal to the distance D₄ less the radiuses R₁, R₂. Further, the entirety of the hook 126 is provided in a common plane in the same way as the hook 26. The hook 126 provides the tendon stripper 120 with similar benefits to the hook 26, including increasing the ease of capturing a tendon, increasing ease of use when the tendon stripper is held in various positions, maintaining tip position during cutting, etc. While two hook arrangements have been illustrated across Figures 1-8, this disclosures extends to other hook arrangements.

It should be understood that terms such as "distal," "proximal," "superior," "inferior," etc., have been used herein for purposes of explanation, and should not be considered otherwise limiting. Terms such as "generally," "substantially," and "about" are not intended to be boundaryless terms, and should be interpreted consistent with the way one skilled in the art would interpret those terms.

Although the different examples have the specific components shown in the illustrations, embodiments of this disclosure are not limited to those particular combinations. It is possible to use some of the components or features from one of the examples in combination with features or components from another one of the examples.

One of ordinary skill in this art would understand that the above-described embodiments are exemplary and non-limiting. That is, modifications of this disclosure would come within the scope of the claims. Accordingly, the following claims should be studied to determine their true scope and content.

## Claims

1. A tendon stripper (20), comprising:
a shaft (24) including a tip (28) provided at a distal end of the shaft; and
a retention structure moveable between an open position and a closed position to capture and retain a tendon (54) relative to the tip (28), wherein the retention structure is provided entirely in a common plane (P) containing a centerline (50) of the shaft, wherein
the retention structure is a hook (26, 126) which is bisected by the common plane, wherein the hook includes a first linear portion (38, 138), a second linear portion (40, 140), and a curved portion (42) connecting the first (38, 138) and second (40, 140) linear portions, wherein the first and second linear portions are substantially parallel to one another along their respective lengths, and the tip (28) tapers to an edge coincident with the common plane and the edge intersects the centerline of the shaft.

2. The tendon stripper (20) according to claim 1,
**characterized in, that**
the tendon stripper (20) further comprising a handle (22), the shaft projecting distally from the handle (22), wherein the handle (22) includes a trigger (30, 34) provided in the common plane (P), and wherein movement of the trigger (30, 34) moves the hook (26, 126) between the open and closed positions.

3. The tendon stripper (20) according to claim 2,
**characterized in, that**
movement of the trigger (30, 34) in the distal direction moves the hook to the open position.

4. The tendon stripper (20) according to claim 1,
**characterized in, that**
the first linear portion (38, 138) is at least partially received within the shaft (24) and mechanically coupled to a trigger (30, 34) provided in a handle (22), the shaft projecting distally from the handle (22), wherein movement of the trigger (30, 34) moves the hook (26, 126) between the open position and the closed position.

5. The tendon stripper (20) according to claim 4,
**characterized in, that**
the second linear portion (40, 140) has a proximal end configured to be received in a socket adjacent the tip (28) when the hook is in the closed position.

6. The tendon stripper (20) according to claim 1,
**characterized in, that**
the curved portion (42) follows an arc defined by a circle having a center between the first (38, 138) and second (40, 140) linear portions, and wherein the first (38, 138) and second (40, 140) linear portions are spaced-apart from one another by a distance substantially equal to a diameter of the circle along substantially their entire respective lengths.

7. The tendon stripper (20) according to claim 1,
**characterized in, that**
the tip (28) spans the entire distance (D4) between the first and second linear portions (38, 40).

## Patentansprüche

1. Sehnenstripper (20), umfassend:
einen Schaft (24), der eine Spitze (28) beinhaltet, die an einem distalen Ende des Schafts vorgesehen ist; und
eine Rückhaltestruktur, die zwischen einer offenen Position und einer geschlossenen Position beweglich ist, um eine Sehne (54) relativ zur Spitze (28) zu fassen und zurückzuhalten, wobei die Rückhaltestruktur vollständig in einer gemeinsamen Ebene (P) vorgesehen ist, die eine Mittellinie (50) des Schafts enthält, wobei
die Rückhaltestruktur ein Haken (26, 126) ist, der durch die gemeinsame Ebene zweigeteilt wird, wobei der Haken einen ersten linearen Abschnitt (38, 138), einen zweiten linearen Abschnitt (40, 140) und einen gekrümmten Abschnitt (42) beinhaltet, der den ersten (38, 138) und zweiten (40, 140) linearen Abschnitt verbindet, wobei der erste und zweite lineare Abschnitt entlang ihrer jeweiligen Längen im Wesentlichen parallel zueinander sind, und die Spitze (28) sich zu einer Kante verjüngt, die mit der gemeinsamen Ebene zusammenfällt, und die Kante die Mittellinie des Schafts schneidet.

2. Sehnenstripper (20) nach Anspruch 1,
**dadurch gekennzeichnet, dass**
der Sehnenstripper (20) ferner einen Griff (22) umfasst, wobei der Schaft distal vom Griff (22) vorsteht, wobei der Griff (22) einen Auslöser (30, 34) beinhaltet, der in der gemeinsamen Ebene (P) vorgesehen ist, und wobei die Bewegung des Auslösers (30, 34) den Haken (26, 126) zwischen der offenen und der geschlossenen Position bewegt.

3. Sehnenstripper (20) nach Anspruch 2,
**dadurch gekennzeichnet, dass**
die Bewegung des Auslösers (30, 34) in der distalen Richtung den Haken in die offene Position bewegt.

4. Sehnenstripper (20) nach Anspruch 1,
**dadurch gekennzeichnet, dass**
der erste lineare Abschnitt (38, 138) zumindest teilweise innerhalb des Schafts (24) aufgenommen und mechanisch mit einem Auslöser (30, 34) gekoppelt ist, der in einem Griff (22) vorgesehen ist, wobei der Schaft distal vom Griff (22) vorsteht, wobei die Bewegung des Auslösers (30, 34) den Haken (26, 126) zwischen der offenen Position und der geschlossenen Position bewegt.

5. Sehnenstripper (20) nach Anspruch 4,
**dadurch gekennzeichnet, dass**
der zweite lineare Abschnitt (40, 140) ein proximales Ende aufweist, das ausgebildet ist, um in einer an die Spitze (28) angrenzenden Buchse aufgenommen zu werden, wenn sich der Haken in der geschlossenen Position befindet.

6. Sehnenstripper (20) nach Anspruch 1,
**dadurch gekennzeichnet, dass**
der gekrümmte Abschnitt (42) entlang eines Bogens verläuft, der durch einen Kreis definiert ist, der eine Mitte zwischen dem ersten (38, 138) und dem zweiten (40, 140) linearen Abschnitt aufweist, und wobei der erste (38, 138) und zweite (40, 140) lineare Abschnitt durch einen Abstand voneinander beabstandet sind, der im Wesentlichen gleich einem Durchmesser des Kreises entlang im Wesentlichen ihrer gesamten jeweiligen Längen ist.

7. Sehnenstripper (20) nach Anspruch 1,
**dadurch gekennzeichnet, dass**
die Spitze (28) sich über den gesamten Abstand (D4) zwischen dem ersten und zweiten linearen Abschnitt (38, 40) erstreckt.

## Revendications

1. Stripper à tendon (20), comprenant :
une tige (24) comprenant une pointe (28) fournie à une extrémité distale de la tige ; et
une structure de rétention mobile entre une position ouverte et une position fermée pour capturer et retenir un tendon (54) par rapport à la pointe (28), dans laquelle la structure de rétention est entièrement fournie dans un plan commun (P) contenant une ligne médiane (50) de la tige, dans laquelle
la structure de rétention est un crochet (26, 126) qui est sectionné en deux par le plan commun, dans lequel le crochet comprend une première partie linéaire (38, 138), une deuxième partie linéaire (40, 140), et une partie incurvée (42) reliant la première (38, 138) et la deuxième (40, 140) parties linéaires, dans lequel les première et deuxième parties linéaires sont sensiblement parallèles l'une par rapport à l'autre le long de leurs longueurs respectives, et la pointe (28) se rétrécit vers un bord coïncidant avec le plan commun et le bord coupe la ligne médiane de la tige.

2. Stripper à tendon (20) selon la revendication 1,
**caractérisé en ce que**
le stripper à tendon (20) comprenant en outre un manche (22), la tige faisant saillie distalement à partir du manche (22), dans lequel le manche (22) comprend un déclencheur (30, 34) fourni dans le plan commun (P), et dans lequel le mouvement du déclencheur (30, 34) déplace le crochet (26, 126) entre les positions ouverte et fermée.

3. Stripper à tendon (20) selon la revendication 2,
**caractérisé en ce que**
un mouvement du déclencheur (30, 34) dans la direction distale déplace le crochet vers la position ouverte.

4. Stripper à tendon (20) selon la revendication 1,
**caractérisé en ce que**
la première partie linéaire (38, 138) est au moins partiellement reçue dans la tige (24) et couplée mécaniquement à un déclencheur (30, 34) fourni dans un manche (22), la tige faisant saillie distalement à partir du manche (22), dans lequel le mouvement du déclencheur (30, 34) déplace le crochet (26, 126) entre la position ouverte et la position fermée.

5. Stripper à tendon (20) selon la revendication 4,
**caractérisé en ce que**
la deuxième partie linéaire (40, 140) a une extrémité proximale configurée pour être reçue dans un logement adjacent à la pointe (28) lorsque le crochet est en position fermée.

6. Stripper à tendon (20) selon la revendication 1,
**caractérisé en ce que**
la partie incurvée (42) suit un arc défini par un cercle ayant un centre entre les première (38, 138) et deuxième (40, 140) parties linéaires, et dans lequel les première (38, 138) et deuxième (40, 140) parties linéaires sont espacées l'une de l'autre par une distance sensiblement égale à un diamètre du cercle sensiblement le long de leurs longueurs respectives entières.

7. Stripper à tendon (20) selon la revendication 1,
**caractérisé en ce que**
la pointe (28) couvre l'entièreté de la distance (D4) entre les première et deuxième parties linéaires (38, 40).
